(19) Europäisches Patentamt
European Patent Office
Office européen des brevets

(11) EP 3 213 813 B1

(12) EUROPEAN PATENT SPECIFICATION

(45) Date of publication and mention of the grant of the patent:
**31.07.2019 Bulletin 2019/31**

(21) Application number: **15855758.7**

(22) Date of filing: **02.11.2015**

(51) Int Cl.:
***B01D 3/00*** *(2006.01)* ***B01D 3/14*** *(2006.01)*
***C07C 7/04*** *(2006.01)* ***C07C 9/15*** *(2006.01)*
***C07C 11/02*** *(2006.01)* ***B01D 3/32*** *(2006.01)*
***B01D 3/42*** *(2006.01)*

(86) International application number:
**PCT/KR2015/011654**

(87) International publication number:
**WO 2016/068677 (06.05.2016 Gazette 2016/18)**

(54) **DISTILLATION METHOD**

DESTILLATIONSVERFAHREN

PROCÉDÉ DE DISTILLATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.10.2014 KR 20140150672**

(43) Date of publication of application:
**06.09.2017 Bulletin 2017/36**

(73) Proprietor: **LG Chem, Ltd.**
**Seoul 07336 (KR)**

(72) Inventors:
- **LEE, Sung Kyu**
**Daejeon 34122 (KR)**
- **SHIN, Joon Ho**
**Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
**WO-A1-99/65582**
**WO-A1-2012/026955**
**KR-A- 950 011 388**
**KR-A- 20070 025 556**
**KR-A- 20140 092 783**
**KR-A- 20140 092 785**
**KR-A- 20140 098 138**
**US-A- 4 177 111**
**US-A- 5 085 740**
**US-A- 5 435 436**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 213 813 B1

## Description

[Technical Field]

The present application relates to a distillation method for isolating and collecting a solvent and an unreacted monomer used in a polymerization process of a polyolefin elastomer.

[Background Art]

Polyolefin elastomers are used as materials having reinforcing properties, such as impact strength and flexural strength, of interior/exterior materials of automobiles. Additionally, polyolefin elastomers are used in various industries, such as the high-tech textiles industry and the sports industry, due to their superior elasticity, toughness, etc.

For example, polyolefin elastomers are polymerized according to a solution polymerization method wherein an olefin monomer is dissolved in a solvent and then polymerization is carried out using a catalyst, and a solvent is collected from a resultant polymerized solution, after which it is subjected to a drying process and packaged. When a polyolefin elastomer is prepared according to such a solution polymerization method, a large amount of solvent is used with respect to the amount of an added olefin monomer, and thus a large amount of energy is consumed in a process of collecting the solvent and an unreacted monomer after polymerization. Conventionally, a solvent and an unreacted monomer were collected from a polymerized solution including the solvent and the unreacted monomer after solution polymerization by means of a distillation device composed of two sequentially connected distillation columns. However, in this process, a large amount of energy is disadvantageously consumed.

WO 2012/026955A1 relates to improved processes and apparatus suitable for energy savings in the distillation of hydrocarbons. More specifically, it concerns processes and apparatus providing energy conservation within an aromatics-processing complex producing xylene isomers.

US 4177111A relates to a process for recovering dimethylacylamides from solutions formed during the production of threads or fibers of acrylonitrile polymerizates or fibers or shaped elements of polyurethanes.

US 5435436A relates to a thermomechanically integrated distillation column and a method for the separation of ethylene from ethane and other close-boiling light hydrocarbons. The column has a plurality of sections operated at successively lower pressures from a high pressure subcritical section to a superatmosphere bottoms product zone. Bottoms liquid from the high pressure and intermediate sections are flashed in respective cooling loops to about the pressure of the section of next lower pressure, vaporized in heat exchange with an overhead condensing zone and introduced to the top stage of next lower pressure section. Vapors from the intermediate sections and the bottoms product zone are compressed in respective compression loops and fed to the bottom stage of the section of the next higher pressure. External refrigerant can be supplied to the overhead condensing zone for trim as needed for control purposes.

WO 99/65582A1 relates generally to improvements in the separation of styrene product from unreacted ethylbenzene following an ethylbenzene-to-styrene dehydrogenation operation.

US 5085740A relates to a process for separating at least one alkene containing 4-10 carbon atoms per molecule from at least one close-boiling alkane containing 4-10 carbon atoms per molecule by extractive distillation of a feed consisting essentially of said at least one alkene and said at least one alkane in the presence of a solvent consisting essentially of a mixture of at least one N-alkyl-2-pyrrolidone, wherein the alkyl group contains 1-3 carbon atoms, and at least one sulfolane compound containing 4-8 carbon atoms per molecule; wherein said process produces an overhead product which contains a smaller volume percentage of said at least one alkene and a larger volume percentage of said at least one alkane than said feed, and a bottoms product which contains said solvent and a larger volume percentage of said at least one alkene and a smaller volume percentage of said at least one alkane than said feed; and wherein said at least one alkene is separated from said solvent and recovered from said bottoms product.

Therefore, there is a need for a process of collecting a solvent and an unreacted monomer to reduce distillation device installation costs and isolate a high-purity compound.

[Disclosure]

[Technical Problem]

The present application is directed to providing a distillation method for isolating and collecting a solvent and an unreacted monomer used in a polymerization process of a polyolefin elastomer with high purity and efficiency.

[Technical Solution]

The present invention provides a distillation method comprising:

introducing a raw material comprising compounds represented by Formulas 1 and 2 below and an isomer of the compound into a first supply port of a first distillation column,
discharging the introduced raw material to each of a first top stream discharged from an upper section of the first distillation column, and first, second and third bottom streams discharged from a lower section of the first distillation column,
introducing the first bottom stream into a second supply port of a second distillation column,
discharging the stream introduced into the second supply port to each of a second top stream discharged from an upper section of the second distillation column; and
fourth and fifth bottom streams discharged from a lower section of the second distillation column;
heat-exchanging the second top stream with the third bottom stream; and
isolating the compound represented by Formula 2 from the upper section of the first distillation column, and the compound represented by Formula 1 from the upper section of the second distillation column,
wherein Equations 1 and 2 below are satisfied:

[Formula 1]

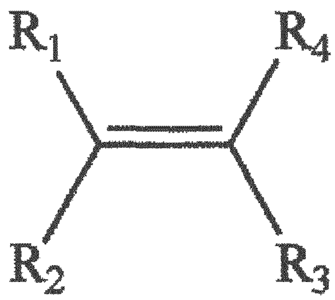

[Formula 2]

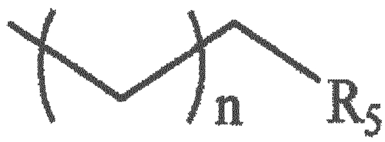

wherein $R_1$ is a $C_4$ to $C_{12}$ alkyl group, and $R_2$ to $R_4$ are each independently hydrogen or a $C_4$ to $C_{12}$ alkyl group, $R_5$ is a $C_1$ to $C_4$ alkyl group, and n is 1 to 4;

[Equation 1]

$$T_{t-2} - T_{b-3} \geq 8\ °C,$$

[Equation 2]

$$P_2/P_1 \geq 3.0,$$

wherein $T_{t-2}$ indicates a temperature of the second top stream, and $T_{b-3}$ indicates a temperature of the third bottom stream, and
$P_1$ indicates a pressure of the upper section of the first distillation column ($kg/cm^2g$), and $P_2$ indicates a pressure of the upper section of the second distillation column ($kg/cm^2g$).

Further embodiments are disclosed in the dependent claims.

Provided is a distillation device. A distillation device may increase economic efficiency of a process by minimizing energy loss occurring in a purification process of an olefin monomer, a solvent, and a raw material including, for example, 1 -octene, iso-octene, and n-hexane used in the polymerization process of the polyolefin elastomer and isolating a product in high purity. In particular, the distillation device provides optimized temperature and pressure for isolating 1-octene, iso-octene, and n-hexane using two distillation units, thus isolating the solvent and the unreacted olefin monomer used in the polyolefin elastomer polymerization process with high purity and efficiency by means of the distillation device.

Hereinafter, the distillation device will be described with reference to the accompanying drawing. The drawing is, however, provided as an exemplary embodiment, .

FIG. 1 exemplarily illustrates a distillation device . As illustrated in FIG. 1, the distillation device includes two distillation units 10 and 20 and a heat exchanger 30. For example, the distillation device includes the first and second distillation units 10 and 20 and the heat exchanger 30. The first distillation unit 10 includes a first distillation column 100, a first condenser 101, a storage tank 102, and a first reboiler 103. The second distillation unit 20 includes a second distillation column 200, a second condenser 201, a storage tank 202, and a second reboiler 203.

The first and second distillation columns 100 and 200 are devices for isolating various ingredients included in a raw material using boiling point differences thereamong. In the distillation device, distillation columns with various shapes may be used, considering ingredients of an introduced raw material or the boiling points of the ingredients to be separated. A distillation column type which may be used in the distillation device is not specifically limited and may be, for example, a distillation column with a general structure as illustrated in FIG. 1 or a dividing wall-type distillation column including a dividing wall therein. In an example, the interiors of the first and second distillation columns 100 and 200 may be divided into upper sections 110 and 210, lower sections 130 and 230, and intermediate sections 120 and 220, as illustrated in FIG. 1. The expression "upper section," as used in the present specification, means a relatively upper portion of each of the first and second distillation columns 100 and 200. For example, when each of the first and second distillation columns 100 and 200 is divided into three parts in a height or length direction thereof, the upper section may be the uppermost section thereof. In addition, the expression "lower section" means a relatively lower portion of each of the first and second distillation columns 100 and 200. For example, when each of the first and second distillation columns 100 and 200 is divided into three parts in a height or length direction thereof, the lower section may be the lowest section thereof. In addition, when each of the first and second distillation columns 100 and 200 is divided into three parts in a height or length direction thereof, the expression "intermediate section" as used in the present specification may mean an intermediate section among the divided sections and a section between the upper section 110 or 210 and the lower section 130 or 220 of each of the first and second distillation columns 100 and 200. In the present specification, the upper section, the lower section and the intermediate section of the distillation column are relative concepts. The tops of the first and second distillation columns 100 and 200 are included in the upper sections, and the bottoms of the first and second distillation columns 100 and 200 are included in the lower sections thereof. Unless mentioned otherwise, "upper section" is synonymous with "the top of a column," and "lower section" is synonymous with "the bottom of a column." The first and second distillation columns 100 and 200 may be distillation columns with a theoretical plate number of 10 to 30, 12 to 28, or 15 to 25. The expression "theoretical plate number" means the number of hypothetical areas or plates, in which two phases such as a vapor phase and a liquid phase establish equilibrium with each other, of the first and second distillation columns 100 and 200.

As illustrated in FIG. 1, the first distillation unit 10 includes the first distillation column 100 and the first condenser 101, the storage tank 102, and the first reboiler 103 connected to the first distillation column 100. The second distillation unit 20 includes the second distillation column 200 and the second condenser 201, the storage tank 202, and the second reboiler 203 connected to the second distillation column 200, as illustrated in FIG. 1. For example, the first distillation column 100, the first condenser 101, the storage tank 102, and the first reboiler 103 may be fluidically connected to each other such that fluid introduced into the first distillation column 100 flows thereinto. The second distillation column 200, the second condenser 201, the storage tank 202, and the second reboiler 203 may be fluidically connected to each other such that fluid introduced into the second distillation column 200 flows thereinto. In addition, the first distillation columns 100 and 200 may be fluidically connected to each other such that a bottom stream of the first distillation columns 100 is introduced into and flows in the intermediate section of the second distillation column 200. The condenser is separately installed on the outside of the distillation column and performs cooling using, for example, a method of bringing a stream discharged from the top of the distillation column into contact with cooling water introduced from the outside. For example, the first condenser 101 of the first distillation column 100 may condense the first top stream $F_{1-2}$ discharged from the upper section 110 of the first distillation column 100, and the second condenser 201 of the second distillation column 200 may condense a second top stream $F_{2-2}$ discharged through the upper section 210 of the second distillation column 200. In addition, the expression "reboiler" means a heating device separately installed on the outside of the distillation column. Alternatively, the reboiler may be a device for reheating and evaporating a stream including ingredients with a high boiling point discharged through the bottom of the distillation column. For example, the first reboiler 103 of the first distillation column 100 may be a device for heating a column bottom stream $F_{1-3}$ discharged through the lower section

130 of the first distillation column 100, and the second reboiler 203 of the second distillation column 200 to be described below may be a device for heating a column bottom stream $F_{2-3}$ discharged through the lower section 230 of the second distillation column 200. "Storage tank" means a tank or a bath for temporarily storing a stream discharged from the distillation column and may be any tank or bath known in the art. For example, the first top stream $F_{1-2}$ discharged from the upper section 110 of the first distillation column 100 is condensed in the first condenser 101, after which it is introduced into the storage tank 102 and stored therein. The second top stream $F_{2-2}$ discharged from the upper section 210 of the second distillation column 200 may be condensed in the second condenser 201 and then introduced into the storage tank 202 and stored therein.

The first distillation column 100 includes a first supply port 121, and the second distillation column 200 includes a second supply port 221. In an embodiment, the first supply port 121 is located at an intermediate section 120 of the first distillation column 100, and the second supply port 221 is located at an intermediate section 220 of the second distillation column 200.

As illustrated in FIG. 1, a raw material $F_{1-1}$ including compounds represented by Formulas 1 and 2 below and the isomer of the compound is introduced into the first supply port 121 of the first distillation column 100:

[Formula 1]

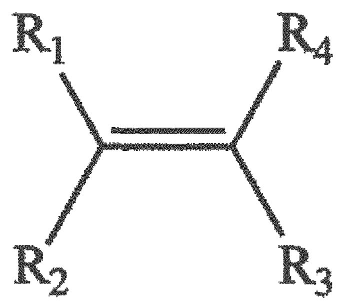

[Formula 2]

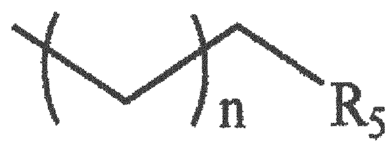

In Formula 1, $R_1$ is a $C_4$ to $C_{12}$ alkyl group, and $R_2$ to $R_4$ are each independently hydrogen or a $C_4$ to $C_{12}$ alkyl group.

In Formula 2, $R_5$ is a $C_1$ to $C_4$ alkyl group, and n is 1 to 4.

In an example, the compound represented by Formula 1 may be at least one selected from the group consisting of, for example, 1-octene, iso-octene, and a mixture thereof, and the compound represented by Formula 2 may be n-hexane, although it is not limited thereto.

In an example, as illustrated in FIG. 1, the raw material $F_{1-1}$ introduced into the first supply port 121 of the first distillation column 100 is introduced into the intermediate section 120 of the first distillation column 100, and a raw material $F_{1-1}$ introduced into the intermediate section 120 of the first distillation column 100 is separately discharged into each of a column top stream discharged through the upper section 110 of the first distillation column 100 and a column bottom stream discharged from the lower section 130 of the first distillation column 100. In this case, the column bottom stream discharged from the lower section 130 of the first distillation column 100 may be separately discharged into at least one stream. For example, the raw material $F_{1-1}$ introduced into the first distillation column 100 may be separately discharged into each of the first top stream $F_{1-2}$, and the first, second and third bottom streams $F_{1-3}$, $F_{1-4}$, and $F_{1-5}$ discharged from the lower section 130 of the first distillation column 100.

The first top stream $F_{1-2}$ discharged from the upper section 110 of the first distillation column 100 is introduced into the first condenser 101, and a portion or all of the first top stream $F_{1-2}$ passing through the first condenser 101 may be refluxed into the upper section 110 of the first distillation column 100 or stored as a product. In an example, a stream discharged from the first condenser 101 may be introduced into the storage tank 102 and stored therein, and then refluxed into the first distillation column 100 or stored as a product. In addition, the first bottom stream $F_{1-3}$ discharged

from the lower section 130 of the first distillation column 100 is introduced into the first reboiler 103. The first bottom stream $F_{1-3}$ passing through the first reboiler 103 may be introduced into the lower section 130 of the first distillation column 100. The first bottom stream $F_{1-3}$ introduced into the first reboiler 103 may be heated by high-pressure stream passing through the first reboiler 103. The amount of this high-pressure stream may be properly controlled by the heat exchanger 30 to be described below. For example, when heat exchange in the heat exchanger 30 is sufficiently carried out, the high-pressure stream may not be used at all. However, when heat exchange is not smoothly carried out due to a large discharge of a raw material or disturbance during a process, isolation efficiency may rapidly decrease. Accordingly, a proper amount of the high-pressure stream may be temporarily used so that robust isolation efficiency can be maintained despite disturbance.

The second bottom stream $F_{1-4}$ discharged from the lower section 130 of the first distillation column 100 is introduced into the second supply port 221 of the second distillation column 200. The second bottom stream $F_{1-4}$ introduced into the second supply port 221 of the second distillation column 200 is introduced into the intermediate section 220 of the second distillation column 200. The second bottom stream $F_{1-4}$ introduced into the intermediate section 220 of the second distillation column 200 is separately discharged into a column top stream discharged from the upper section 210 of the second distillation column 200 and a column bottom stream discharged from the lower section 230 of the second distillation column 200. In this case, the column bottom stream discharged from the lower section 230 of the second distillation column 200 may be separately discharged into at least one stream. For example, a stream introduced into the second distillation column 200 may be separately discharged into the second top stream $F_{2-2}$, and a fourth bottom stream $F_{2-3}$ and a fifth bottom stream $F_{2-4}$ discharged from the lower section 230 of the second distillation column 200.

The fourth bottom stream $F_{2-3}$ discharged from the lower section 230 of the second distillation column 200 is introduced into the second reboiler 203. The fourth bottom stream $F_{2-3}$ passing through the second reboiler 203 is introduced into the lower section 230 of the second distillation column 200, and the fifth bottom stream $F_{2-4}$ discharged from the lower section 230 of the second distillation column 200 may be stored as a product.

The third bottom stream $F_{1-5}$ discharged from the lower section 130 of the first distillation column 100 and the second top stream $F_{2-2}$ discharged from the upper section 210 of the second distillation column 200 are introduced into the heat exchanger 30. The "heat exchanger" is separately installed on the outside of the distillation column and performs heat exchange such that heat transfer between two fluid streams, the temperatures of which are different, is smoothly carried out. For example, the heat exchanger 30 may allow heat exchange between the third bottom stream $F_{1-5}$ discharged from the lower section 130 of the first distillation column 100 and the second top stream $F_{2-2}$ discharged from the upper section 210 of the second distillation column 200. In the distillation device, the third bottom stream $F_{1-5}$ with a high boiling point discharged from the lower section 130 of the first distillation column 100 and the second top stream $F_{2-2}$ with a low boiling point discharged from the upper section 210 of the second distillation column 200 exchange heat with each other in the heat exchanger 30, thereby reducing energy required in condensation and heating processes in which the condenser or the reboiler is used, and isolating and collecting 1-octene/iso-octene and n-hexane in high purity.

The heat exchanger 30 may be directly or indirectly connected to pipes through which the third bottom stream $F_{1-5}$ of the first distillation column 100 and the second top stream $F_{2-2}$ of the second distillation column 200 pass. In an example, the heat exchanger 30 is directly connected to the pipes through which the third bottom stream $F_{1-5}$ of the first distillation column 100 and the second top stream $F_{2-2}$ of the second distillation column 200 pass, whereby heat exchange between the third bottom stream $F_{1-5}$ and the second top stream $F_{2-2}$ may be efficiently performed.

Heat exchange between the third bottom stream $F_{1-5}$ and the second top stream $F_{2-2}$ introduced into the heat exchanger 30 is carried out, the third bottom stream $F_{1-5}$ passing through the heat exchanger 30 is refluxed into the lower section 130 of the first distillation column 100, the second top stream $F_{2-2}$ passing through the heat exchanger 30 is introduced into the second condenser 201, and a portion or all of the second top stream $F_{2-2}$ passing through the second condenser 201 may be refluxed into the upper section 210 of the second distillation column 200 or stored as a product. In an example, a stream discharged from the second condenser 201 is introduced in the storage tank 202 and stored therein. Subsequently, the stored stream may be refluxed into the second distillation column 200 or stored as a product.

In the heat exchanger 30, the third bottom stream $F_{1-5}$ may exchange heat with the second top stream $F_{2-2}$ before the third bottom stream $F_{1-5}$ is refluxed into the first distillation column 100, and the second top stream $F_{2-2}$ may exchange heat with the third bottom stream $F_{1-5}$ before the second top stream $F_{2-2}$ is introduced into the second condenser 201. For example, the second top stream $F_{2-2}$ including an ingredient with a low boiling point discharged from the upper section 210 of the second distillation column 200 transits the heat exchanger 30 before being refluxed into the upper section 210 of the second distillation column 200. At this time, heat is supplied to the heat exchanger 30. Accordingly, the second top stream $F_{2-2}$ discharged from the second distillation column 200 may be refluxed at a relatively low temperature in the second distillation column 200. Accordingly, the quantity of heat necessary to condense the second top stream $F_{2-2}$ discharged from the upper section 210 of the second distillation column 200 may be decreased, and costs necessary for the condensation process may be reduced by decreasing the amount of cooling water used in the

condensation process in which the second condenser 201 is used. In addition, the third bottom stream $F_{1-5}$ is a stream including an ingredient with a high boiling point discharged from the lower section 130 of the first distillation column 100, and transits the heat exchanger 30 before being refluxed into the lower section 130 of the first distillation column 100. At this time, heat transferred by the second top stream $F_{2-2}$ may be supplied to the third bottom stream $F_{1-5}$. Accordingly, the second top stream $F_{2-2}$ supplies heat to the lower section 130 of the first distillation column 100 and thus the amount of the stream used in the first reboiler 103 in order to heat the first bottom stream $F_{1-3}$ discharged from the lower section 130 of the first distillation column 100 is reduced, thereby reducing costs.

Hereinafter, a process of isolating an olefin monomer, a solvent, and a raw material such as, for example, 1-octene/iso-octene, n-hexane, etc. used in polymerizing the polyolefin elastomer by means of the distillation method according to an embodiment of the present application will be described in detail.

In an example, the raw material $F_{1-1}$ including 1-octene or iso-octene represented by Formula 1 or a mixture thereof, and n-hexane represented by Formula 2 as main ingredients is introduced into the first supply port 121 of the first distillation column 100.

In this case, a stream including a large amount of n-hexane with a relatively low boiling point among ingredients that are included in the raw material $F_{1-1}$ introduced into the first supply port 121 may be discharged as the first top stream $F_{1-2}$ from the upper section 110 of the first distillation column 100, and a stream including a large amount of 1-octene or iso-octene with a relatively high boiling point may be discharged as the first top stream $F_{1-2}$, and the first, second and third bottom streams $F_{1-3}$, $F_{1-4}$, and $F_{1-5}$ from the lower section 130 of the first distillation column 100. The first top stream $F_{1-2}$ discharged through the upper section 110 of the first distillation column 100 is introduced into the storage tank 102 via the first condenser 101. A portion of the stream discharged from the storage tank 102 is refluxed into the upper section 110 of the first distillation column 100, and a portion of the remainder of the stream may be stored as a product. The product may be high-purity n-hexane. Meanwhile, the first bottom stream $F_{1-3}$ discharged from the lower section 130 of the first distillation column 100 may be refluxed into the lower section 130 of the first distillation column 100 via the first reboiler 103, and the second bottom stream $F_{1-4}$ may be introduced into the second supply port 221 of the second distillation column 200. In addition, the third bottom stream $F_{1-5}$ may exchange heat with the second top stream $F_{2-2}$ of the second distillation column 200 in the heat exchanger 30, and then be refluxed into the lower section 130 of the first distillation column 100.

In addition, the second bottom stream $F_{1-4}$ introduced into the second supply port 221 includes 1-octene and/or iso-octene and ingredients with a high boiling point. Accordingly, a stream including a large amount of 1-octene and/or iso-octene with a relatively low boiling point among ingredients included the second bottom stream $F_{1-4}$ may be discharged as the second top stream $F_{2-2}$ from the upper section 210 of the second distillation column 200, and a stream including ingredients with a relatively high boiling point may be discharged as the fourth and fifth bottom streams $F_{2-3}$ and $F_{2-4}$ from the lower section 230 of the second distillation column 200. The discharged second top stream $F_{2-2}$ exchanges heat with the third bottom stream $F_{1-5}$ of the first distillation column 100 in the heat exchanger 30 and is then introduced into the storage tank 202 via the second condenser 201. A portion of the stream discharged from the storage tank 202 may be refluxed into the upper section 210 of the second distillation column 200, and another portion of the stream may be stored as a product. The product may be high-purity 1-octene and/or iso-octene. In addition, a stream including an ingredient with a relatively high boiling point among ingredients included in the second top stream $F_{2-2}$ may be discharged as the fourth and fifth bottom streams $F_{2-3}$ and $F_{2-4}$ from the lower section 230 of the second distillation column 200. The fourth bottom stream $F_{2-3}$ may be refluxed into the lower section 230 of the second distillation column 200 via the second reboiler 203 and the fifth bottom stream $F_{2-4}$ may be utilized as fuel. The fifth bottom stream $F_{2-4}$ may be, for example, an octene-based ingredient and/or an ingredient with a high boiling point.

In the present specification, the expression "stream including an ingredient with a low boiling point" means a stream including a large amount of an ingredient with a relatively low boiling point of the raw material stream $F_{1-1}$ including an ingredient with a low boiling point and an ingredient with a high boiling point. For example, streams including ingredients with low boiling points are streams discharged from the upper sections 110 and 210 of the first and second distillation columns 100 and 200. In addition, the expression "stream including an ingredient with a high boiling point" means a stream including a large amount of an ingredient with a relatively high boiling point of the raw material stream $F_{1-1}$ including an ingredient with a low boiling point and an ingredient with a high boiling point. For example, a stream including an ingredient with a high boiling point is a stream including a large amount of an ingredient with a relatively high boiling point discharged from the lower sections 130 and 230 of the first and second distillation columns 100 and 200. The expression "stream including a large amount of an ingredient" means a stream in which the content of each of an ingredient with a low boiling point included in a stream discharged from the upper sections 110 and 210 of the first and second distillation columns 100 and 200 and an ingredient with a high boiling point included in a stream discharged from the lower sections 130 and 230 of the first and second distillation columns 100 and 200 is higher than the content of each of the ingredient with a low boiling point and the ingredient with a high boiling point included in the raw material $F_{1-1}$. For example, the content of each of the ingredient with a low boiling point included in the first top stream $F_{1-2}$ of the first distillation column 100 and the ingredient with a low boiling point included in the second top stream $F_{2-2}$ of the

second distillation column 200 may be 50% by weight or more, 80% by weight or more, 90% by weight or more, 95% by weight or more or 99% by weight or more. Alternatively, the contents of an ingredient with a high boiling point included in each of the first top stream $F_{1-2}$, and the first, second and third bottom streams $F_{1-3}$, $F_{1-4}$, and $F_{1-5}$ of the first distillation column 100 and an ingredient with a high boiling point included in each of the fourth and fifth bottom streams $F_{2-3}$ and $F_{2-4}$ of the second distillation column 200 may each be 50% by weight or more, 80% by weight or more, 90% by weight or more, 95% by weight or more, or 99% by weight or more.

In an example, a portion of the fifth bottom stream $F_{2-4}$ discharged from the lower section 230 of the second distillation column 200 may be introduced into the lower section 130 of the first distillation column 100, for example, a 13th to 23rd plate of the first distillation column 100 with a theoretical plate number of 15 to 25. Accordingly, 1-octene and/or iso-octene that may remain in the fifth bottom stream $F_{2-4}$ may be supplied to the lower section 130 of the first distillation column 100, and thereby 1-octene and/or iso-octene with higher purity may be prepared. In this case, a ratio of the discharge rate (kg/hr) of the stream introduced into the lower section 130 of the first distillation column 100 to a discharge rate (kg/hr) of the fifth bottom stream $F_{2-4}$ discharged from the lower section 230 of the second distillation column 200 may be 1:0.8 to 1:0.95. By controlling a discharge ratio of the stream introduced into the lower section 130 of the first distillation column 100 within this range, 1-octene and/or iso-octene with higher purity may be prepared.

In an embodiment, the distillation method of the present application satisfies Equation 1 below.

[Equation 1]

$$T_{t-2} - T_{b-3} \geq 8\ °C$$

wherein $T_{t-2}$ indicates the temperature of the second top stream $F_{2-2}$, and $T_{b-3}$ indicates the temperature of the third bottom stream $F_{1-5}$.

When the distillation method of the present application satisfies Equation 1, the compounds represented by Formulas 1 and 2, particularly 1-octene/iso-octene and n-hexane, may be isolated with superior efficiency and high purity using the distillation device with the aforementioned series structure. That is, by controlling the distillation device such that a temperature difference between the second top stream $F_{2-2}$ and the third bottom stream $F_{1-5}$ satisfies Equation 1, heat exchange efficiency between the second top stream $F_{2-2}$ and the third bottom stream $F_{1-5}$ may be maximized. Accordingly, the compounds represented by Formulas 1 and 2, particularly 1-octene, iso-octene, or a mixture thereof and n-hexane, may be isolated with superior efficiency and high purity.

In an example, as long as a temperature difference between the second top stream $F_{2-2}$ discharged from the upper section 210 of the second distillation column 200 and the third bottom stream $F_{1-5}$ discharged from the lower section 130 of the first distillation column 100 satisfies Equation 1, there is no specific limitation. For example, the temperature difference may be 8 °C or more, 9 °C or more, 10 °C or more, 13 °C or more, or 15 °C or more. Since heat exchange efficiency is superior when the temperature difference between the second top stream $F_{2-2}$ discharged from the upper section 210 of the second distillation column 200 and the third bottom stream $F_{1-5}$ discharged from the lower section 130 of the first distillation column 100 increases, the maximum value of the temperature difference value is not specifically limited. For example, a temperature difference between the second top stream $F_{2-2}$ discharged from the upper section 210 of the second distillation column 200 and the third bottom stream $F_{1-5}$ discharged from the lower section 130 of the first distillation column 100 may be 100 °C or less, considering process efficiency.

In an example, the distillation method of the present application satisfies Equation 2 below:

[Equation 2]

$$P_2/P_1 \geq 3.0$$

wherein $P_1$ indicates the pressure ($kg/cm^2g$) of the upper section 110 of the first distillation column 100, and $P_2$ indicates the pressure ($kg/cm^2g$) of the upper section 210 of the second distillation column 200.

When the distillation method of the present application satisfies Equation 1, 1-octene or iso-octene represented by Formula 1 or a mixture thereof and n-hexane represented by Formula 1 may be isolated in superior efficiency and high purity using the distillation device with the aforementioned series structure. That is, by controlling the distillation device such that a ratio of the pressure of the upper section 210 of the second distillation column 200 to the pressure of the upper section 110 of the first distillation column 100 satisfies Equation 2, heat exchange efficiency between the second top stream $F_{2-2}$ and the third bottom stream $F_{1-5}$ may be maximized. Accordingly, 1-octene or iso-octene represented by Formula 1 or a mixture thereof and n-hexane represented by Formula 2 may be isolated with superior efficiency and high purity.

For example, in order to increase the heat exchange efficiency of the heat exchanger 30, the interior temperature of the first distillation column 100 may be kept lower than the interior temperature of the second distillation column 200, and thus the pressure of the upper section 110 of the first distillation column 100 may be kept lower than that of the upper section 210 of the second distillation column 200.

In an example, as long as a ratio of the pressure of the upper section 210 of the second distillation column 200 to the pressure of the upper section 110 of the first distillation column 100 satisfies Equation 2, there is no specific limitation. For example, the ratio may be 3.0 or more, 4.0 or more, 5.0 or more, or 8.0 or more. Since heat exchange efficiency improves when the ratio of the pressure of the upper section 210 of the second distillation column 200 to the pressure of the upper section 110 of the first distillation column 100 increases, the maximum value of the ratio is not specifically limited. For example, the ratio of the pressure of the upper section 210 of the second distillation column 200 to the pressure of the upper section 110 of the first distillation column 100 may be 200 or less, or 100 or less, considering process efficiency.

The temperature of the second top stream $F_{2-2}$ discharged from the upper section 210 of the second distillation column 200 is not specifically limited as long as Equation 1 is satisfied. The temperature may be 125 to 170 °C, for example, 130 °C to 168 °C or 140 °C to 165 °C. In addition, the temperature of the third bottom stream $F_{1-5}$ discharged from the lower section 130 of the first distillation column 100 is not specifically limited as long as Equation 1 is satisfied. The temperature may be 120 °C to 145 °C, for example, 122 °C to 140 °C or 125 °C to 135 °C. In this case, the pressure of the upper section 110 of the first distillation column 100 is not specifically limited as long as Equation 2 is satisfied. The pressure may be 0.05 to 0.2 kg/cm$^2$g, 0.08 to 0.18 kg/cm$^2$g, or 0.1 to 0.16 kg/cm$^2$g. In addition, the pressure of the upper section 210 of the second distillation column 200 is not specifically limited as long as Equation 2 is satisfied. The pressure may be 1.0 to 2.0 kg/cm$^2$g, 1.1 to 1.8 kg/cm$^2$g, or 1.2 to 1.6 kg/cm$^2$g.

In an example, the temperature of the upper section 110 of the first distillation column 100 may be 60 °C to 80 °C, for example, 62 °C to 78 °C or 64 °C to 76 °C, and the temperature of the lower section 130 of the first distillation column 100 may be 120 °C to 145 °C, for example, 122 °C to 140 °C or 124 °C to 135 °C, although the present application is not limited thereto. In this case, the temperature of the upper section 210 of the second distillation column 200 may be 125 °C to 170 °C, for example, 130 °C to 168 °C or 140 °C to 165 °C, and the temperature of the lower section 230 of the second distillation column 200 may be 130 °C to 180 °C, for example, 135 °C to 175 °C or 140 °C to 170 °C, although the present application is not limited thereto.

The present disclosure relates to a distillation method of isolating the solvent used in the polyolefin elastomer polymerization process from the unreacted olefin monomer.

The distillation method according to an exemplary embodiment of the present application may be carried out using the aforementioned distillation device, and thus the same content as the description of the aforementioned distillation device is omitted.

In an embodiment, the distillation method of the present application includes a) a step of introducing the raw material $F_{1-1}$ including the compounds represented by Formulas 1 and 2 below into the first supply port 121 of the first distillation column 100; b) a step of discharging the introduced raw material $F_{1-1}$ to each of the first top stream $F_{1-2}$ discharged from the upper section 110 of the first distillation column 100; and the first top stream $F_{1-2}$, and the first, second and third bottom streams $F_{1-3,}$ $F_{1-4}$, and $F_{1-5}$ discharged from the lower section 130 of the first distillation column 100; c) a step of introducing the first bottom stream $F_{1-3}$ into the second supply port 221 of the second distillation column 200; d) a step of discharging the stream introduced into the second supply port 221 to each of the second top stream $F_{2-2}$ discharged from the upper section 210 of the second distillation column 200, and the fourth and fifth bottom streams $F_{2-3}$ and $F_{2-4}$ discharged from the lower section 230 of the second distillation column 200; e) a step of exchanging heat between the second top stream $F_{2-2}$ and the third bottom stream $F_{1-5}$; and f) a step of isolating the compound represented by Formula 2 from the upper section 110 of the first distillation column 100, and the compound represented by Formula 1 from the upper section 210 of the second distillation column 200:

[Formula 1]

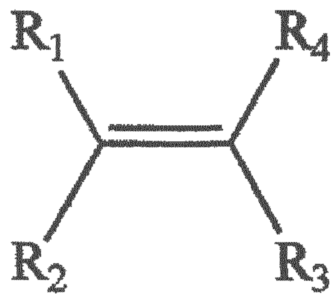

[Formula 2]

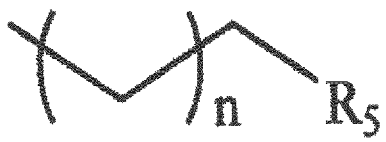

In Formula 1, $R_1$ is a $C_4$ to $C_{12}$ alkyl group, and $R_2$ to $R_4$ are each independently hydrogen or a $C_4$ to $C_{12}$ alkyl group.

In Formula 2, $R_5$ is a $C_1$ to $C_4$ alkyl group, and n is 1 to 4.

The distillation method may be carried out using the aforementioned distillation device. Description of the distillation device is the same as that given above, and is thus omitted.

As described above, steps a) to f) are each independently organically connected, and thus boundaries therebetween are not clearly divided according to chronological order. Each of steps a) to f) may be carried out sequentially or independently at the same time.

The distillation method satisfies Equations 1 and 2 below. Description thereof is the same as that given above, and is thus omitted.

[Equation 1]

$$T_{t-2} - T_{b-3} \geq 8\ °C$$

[Equation 2]

$$P_2/P_1 \geq 3.0$$

In Equation 1, $T_{t-2}$ indicates the temperature of the second top stream $F_{2-2}$, and $T_{b-3}$ indicates the temperature of the third bottom stream $F_{1-5}$.

In Equation 2, $P_1$ indicates the pressure ($kg/cm^2g$) of the upper section 110 of the first distillation column 100, and $P_2$ indicates the pressure ($kg/cm^2g$) of the upper section 210 of the second distillation column 200.

[Advantageous Effects]

As apparent from the foregoing, the distillation method of the present application can minimize energy loss occurring in a purification process of the olefin monomer, the solvent, and the raw material including, for example, 1-octene/iso-octene and n-hexane, used in a polymerization process of the polyolefin elastomer, and can increase economic

efficiency by isolating a high-purity product.

[Description of Drawings]

FIG 1 exemplarily illustrates a distillation device.

[Modes of the Invention]

Now, the present invention will be described in more detail with reference to examples according to the present invention and comparative examples. These examples are provided for illustrative purposes only..

Example 1

1-Octene, iso-octene, and n-hexane were isolated by means of a distillation device illustrated in FIG. 1. In particular, a raw material including 1-octene, iso-octene, and n-hexane was introduced into a first supply port located at a 15$^{th}$ plate of a first distillation column with a theoretical plate number of 21.

A portion of a first top stream discharged from an upper section of the first distillation column was refluxed into the upper section of the first distillation column via a first condenser. A portion of the remainder of the first top stream was isolated as a product including n-hexane and stored. A portion of a first bottom stream discharged from a lower section of the first distillation column was refluxed into the lower section of the first distillation column via a first reboiler. A second bottom stream discharged from the lower section of the first distillation column was introduced into a second supply port located at a 7$^{th}$ plate of a second distillation column with a theoretical plate number of 12. A third bottom stream discharged from the lower section of the first distillation column was introduced into a heat exchanger and heat-exchanged with a second top stream of the second distillation column introduced into the heat exchanger, and then was refluxed into the lower section of the first distillation column via the heat exchanger. In this case, operation pressure of the upper section of the first distillation column was adjusted to 0.16 kg/cm$^2$g and an operation temperature thereof was adjusted to 75 °C. An operation temperature of the lower section of the first distillation column was adjusted to 130 °C.

Meanwhile, the second top stream discharged from an upper section of the second distillation column was introduced into the heat exchanger and heat-exchanged with the third bottom stream. Subsequently, a portion of the second top stream having passed through the heat exchanger and the second condenser was refluxed into the upper section of the second distillation column, and a portion of the remainder of the second top stream was isolated as an octene-based product including 1-octene and iso-octene. In this case, the purity of each of the 1-octene and iso-octene was 94%. A fourth bottom stream discharged from a lower section of the second distillation column was refluxed into the lower section of the second distillation column via a second reboiler, and a fifth bottom stream discharged from the lower section of the second distillation column was isolated as a product for fuel including some octene-based materials and an ingredient with a high boiling point. In this case, operation pressure of the upper section of the second distillation column was adjusted to 1.4 kg/cm$^2$g, and an operation temperature thereof was adjusted to 155 °C. An operation temperature of the lower section of the second distillation column was adjusted to 160 °C.

In isolating 1-octene, iso-octene, and n-hexane by means of the distillation method of Example 1, a used energy amount, a recovery amount, a reduction amount, a reduction rate, and the purities of a mixture of 1-octene and iso-octene, and an n-hexane product were as summarized in Table 1 below.

**Example 2**

1-Octene, iso-octene, and n-hexane were isolated in the same manner as in Example 1, except that the operation conditions of the first and second distillation columns were changed as disclosed in Table 1 below.

In isolating 1-octene, iso-octene, and n-hexane by means of a distillation method of Example 2, a used energy amount, a recovery amount, a reduction amount, a reduction rate, and the purities of a mixture of 1-octene and iso-octene, and an n-hexane product were as summarized in Table 1 below.

**Example 3**

1-Octene, iso-octene, and n-hexane were isolated in the same manner as in Example 1, except that the operation conditions of the first and second distillation columns were changed as disclosed in Table 1 below.

In isolating 1-octene, iso-octene, and n-hexane by means of a distillation method of Example 3, a used energy amount, a recovery amount, a reduction amount, a reduction rate, and the purities of a mixture of 1-octene and iso-octene, and an n-hexane product were as summarized in Table 1 below.

**Example 4**

1-Octene, iso-octene, and n-hexane were isolated in the same manner as in Example 1, except that the operation conditions of the first and second distillation columns were changed as disclosed in Table 1 below.

In isolating 1-octene, iso-octene, and n-hexane by means of a distillation method of Example 4, a used energy amount, a recovery amount, a reduction amount, a reduction rate, and the purities of a mixture of 1-octene and iso-octene, and an n-hexane product were as summarized in Table 1 below.

**Comparative Example 1**

1-Octene, iso-octene, and n-hexane were isolated in the same manner as in Example 1, except that the operation conditions of the first and second distillation columns were changed as disclosed in Table 2 below.

In isolating 1-octene, iso-octene, and n-hexane by means of a distillation method of Comparative Example 1, a used energy amount, a recovery amount, a reduction amount, a reduction rate, and the purities of a mixture of 1-octene and iso-octene, and an n-hexane product were as summarized in Table 2 below.

**Comparative Example 2**

1-Octene, iso-octene, and n-hexane were isolated in the same manner as in Example 1, except that the operation conditions of the first and second distillation columns were changed as disclosed in Table 2 below.

In isolating 1-octene, iso-octene, and n-hexane by means of a distillation method of Comparative Example 2, a used energy amount, a recovery amount, a reduction amount, a reduction rate, and the purities of a mixture of 1-octene and iso-octene, and n-hexane were as summarized in Table 2 below.

**Comparative Example 3**

1-Octene, iso-octene, and n-hexane were isolated in the same manner as in Example 1, except that the operation conditions of the first and second distillation columns were changed as disclosed in Table 2 below.

In isolating 1-octene, iso-octene, and n-hexane by means of a distillation method of Comparative Example 3, a used energy amount, a recovery amount, a reduction amount, a reduction rate, and the purities of a mixture of 1-octene and iso-octene, and an n-hexane product were as summarized in Table 2 below.

**Comparative Example 4**

1-Octene, iso-octene, and n-hexane were isolated in the same manner as in Example 1, except that the operation conditions of the first and second distillation columns were changed as disclosed in Table 3 below.

In isolating 1-octene, iso-octene, and n-hexane by means of a distillation method of Comparative Example 4, a used energy amount, a recovery amount, a reduction amount, a reduction rate, and the purities of a mixture of 1-octene and iso-octene, and an n-hexane product were as summarized in Table 3 below.

**Comparative Example 5**

1-Octene, iso-octene, and n-hexane were isolated in the same manner as in Example 1, except that the operation conditions of the first and second distillation columns were changed as disclosed in Table 3 below.

In isolating 1-octene, iso-octene, and n-hexane by means of a distillation method of Comparative Example 5, a used energy amount, a recovery amount, a reduction amount, a reduction rate, and the purities of a mixture of 1-octene and iso-octene, and an n-hexane product were as summarized in Table 3 below.

[Table 1]

| | | Example 1 | Example 2 | Example 3 | Example 4 |
|---|---|---|---|---|---|
| Pressure of upper section (kg/cm$^2$g) | First distillation column | 0.16 | 0.13 | 0.15 | 0.05 |
| | Second distillation column | 1.4 | 1.1 | 1.2 | 1.2 |

(continued)

| | | Example 1 | Example 2 | Example 3 | Example 4 |
|---|---|---|---|---|---|
| Column temperature (°C) (upper section/ lower section) | First distillation column | 75/130 | 72/126 | 74/130 | 71/127 |
| | Second distillation column | 155/160 | 149/155 | 151/157 | 151/157 |
| Energy (GJ/hr) (Gcal/hr) | First distillation column | 2.93 (0.7) | 3.47 (0.83) | 3.68 (0.88) | 3.09 (0.74) |
| | Second distillation column | 3.05 (0.73) | 3.09 (0.74) | 3.09 (0.74) | 3.09 (0.74) |
| | Recovery amount | 2.93 (0.7) | 2.63 (0.63) | 2.59 (0.62) | 2.80 (0.67) |
| | Total | 3.05 (0.73) | 3.93 (0.94) | 4.18 (1.00) | 3.39 (0.81) |
| | Reduction amount | 2.46 (0.59) | 1.59 (0.38) | 1.33 (0.32) | 2.13 (0.51) |
| | Energy reduction rate (%) | 2.93 (0.7) | 3.47 (0.83) | 3.68 (0.88) | 3.09 (0.74) |
| Product purity (%) | 1-Octene+iso-octene | 94 | 94 | 94 | 94 |
| | n-Hexane | 99.3 | 99.3 | 99.3 | 99.3 |

[Table 2]

| | | Comparative Example 1 | Comparative Example 2 | Comparative Example 3 |
|---|---|---|---|---|
| Pressure of upper section ($kg/cm^2g$) | First distillation column | 0.3 | 0.75 | 0.75 |
| | Second distillation column | 0.2 | 1.2 | 2.0 |
| Column temperature (°C) (upper section/lower section) | First distillation column | 80/135 | 88/144 | 88/144 |
| | Second distillation column | 130/135 | 151/157 | 166/172 |

(continued)

| | | Comparative Example 1 | Comparative Example 2 | Comparative Example 3 |
|---|---|---|---|---|
| Energy (GJ/hr) (Gcal/hr) | First distillation column | 3.05 (0.73) | 7.24 (1.73) | 7.24 (1.73) |
| | Second distillation column | 2.46 (0.59) | 3.09 (0.74) | 4-01 (0-96) |
| | Recovery amount | - | 1.88 (0.45) | 1.88 (0.45) |
| | Total | 5.52 (1.32) | 8.45 (2.02) | 9.37 (2.24) |
| | Reduction amount | - | - | - |
| | Energy reduction rate (%) | - | - | - |
| Product purity (%) | 1-Octene+iso-octene | 94 | 94 | 94 |
| | n-Hexane | 99.3 | 99.3 | 99.3 |

[Table 3]

| | | Comparative Example 4 | Comparative Example 5 |
|---|---|---|---|
| Pressure of upper section ($kg/cm^2g$) | First distillation column | 0.75 | 0.68 |
| | Second distillation column | 1.23 | 1.15 |
| Column temperature (°C) (upper section/lower section) | First distillation column | 88/144 | 86/143 |
| | Second distillation | 152/160 | 151/159 |
| Energy (GJ/hr) (Gcal/hr) | First distillation column | 7.24 (1.73) | 6.61 (1.58) |
| | Second distillation column | 3.22 (0.77) | 2.93 (0.70) |
| | Recovery amount | 1.88 (0.45) | 1.88 (0.45) |
| | Total | 8.58 (2.05) | 7.66 (1.83) |
| | Reduction amount | - | - |
| | Energy reduction rate (%) | - | - |
| Product purity (%) | 1-Octene+iso-octene | 94 | 94 |
| | n-Hexane | 99.3 | 99.3 |

As shown in Tables 1 to 3, it can be confirmed that, when 1-octene, iso-octene, and n-hexane are isolated according to each of Examples 1 to 4, a total energy consumption amount is greatly decreased, compared to the

comparative examples. Accordingly, when the raw material is isolated by means of the distillation method according to each of Examples 1 to 4 of the present application, energy reduction effect up to 44.7% can be achieved, compared to the cases in which the distillation devices according to the comparative examples are used.

In addition, it can be confirmed that, as shown in the examples and the comparative examples, 1-octene, iso-octene, and n-hexane can be isolated with high purity and efficiency by controlling a temperature difference between the lower section of the first distillation column and the upper section of the second distillation column and the pressures of the upper sections of the first and second distillation columns within a specific range.

## Claims

**1.** A distillation method, comprising:

introducing a raw material comprising compounds represented by Formulas 1 and 2 below and an isomer of the compound into a first supply port of a first distillation column,
discharging the introduced raw material to each of a first top stream discharged from an upper section of the first distillation column, and first, second and third bottom streams discharged from a lower section of the first distillation column,
introducing the first bottom stream into a second supply port of a second distillation column,
discharging the stream introduced into the second supply port to each of a second top stream discharged from an upper section of the second distillation column; and fourth and fifth bottom streams discharged from a lower section of the second distillation column;
heat-exchanging the second top stream with the third bottom stream; and
isolating the compound represented by Formula 2 from the upper section of the first distillation column, and the compound represented by Formula 1 from the upper section of the second distillation column,
wherein Equations 1 and 2 below are satisfied:

[Formula 1]

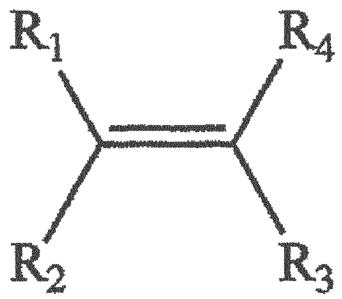

[Formula 2]

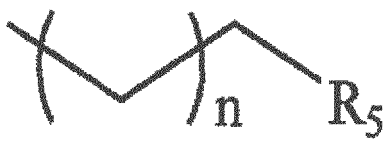

wherein $R_1$ is a $C_4$ to $C_{12}$ alkyl group, and $R_2$ to $R_4$ are each independently hydrogen or a $C_4$ to $C_{12}$ alkyl group, $R_5$ is a $C_1$ to $C_4$ alkyl group, and n is 1 to 4;

[Equation 1]

$$T_{t-2}-T_{b-3} \geq 8\ ^{\circ}C,$$

[Equation 2]

$$P_2/P_1 \geq 3.0,$$

wherein $T_{t-2}$ indicates a temperature of the second top stream, and $T_{b-3}$ indicates a temperature of the third bottom stream, and
$P_1$ indicates a pressure of the upper section of the first distillation column ($kg/cm^2g$), and $P_2$ indicates a pressure of the upper section of the second distillation column ($kg/cm^2g$).

**2.** The distillation method according to claim 1, wherein the compound represented by Formula 1 is at least one selected from the group consisting of 1-octene, iso-octene and a mixture thereof, and the compound represented by Formula 2 is n-hexane.

**3.** The distillation method according to claim 2, wherein a content of n-hexane in the first top stream is 90% or more, and a content of 1-octene, iso-octene, or a mixture thereof in the second top stream is 90% or more.

**4.** The distillation method according to claim 1, wherein a portion of the second top stream is introduced into the heat exchanger, a portion of a remainder of the second top stream is introduced into the second condenser, a portion of the second top stream passing through the heat exchanger is introduced into the second condenser, and a portion or all of the second top stream passing through the second condenser is introduced into the upper section of the second distillation column.

**5.** The distillation method according to claim 1, wherein a pressure of the upper section of the first distillation column is 0.05 to 0.2 $kg/cm^2g$.

**6.** The distillation method according to claim 1, wherein a pressure of the upper section of the second distillation column is 1.0 to 2.0 $kg/cm^2g$.

**7.** The distillation method according to claim 1, wherein a temperature of the upper section of the first distillation column is 60 to 80 °C.

**8.** The distillation method according to claim 1, wherein a temperature of the lower section of the first distillation column is 120 to 145 °C.

**9.** The distillation method according to claim 1, wherein a temperature of the upper section of the second distillation column is 125 to 170 °C.

**10.** The distillation method according to claim 1, wherein a temperature of the lower section of the second distillation column is 130 to 180 °C.

**11.** The distillation method according to claim 1, wherein the compound represented by Formula 2 is a solvent used in polymerization of a polyolefin elastomer.

## Patentansprüche

**1.** Destillationsverfahren, umfassend:

Einführen eines Rohmaterials umfassend Verbindungen, die durch Formeln 1 und 2 unten dargestellt sind, und ein Isomer der Verbindung in eine erste Lieferöffnung einer ersten Destillationssäule,
Abgeben des eingeführten Rohmaterials an jeden eines ersten Oberstroms, der von einem oberen Abschnitt der ersten Destillationssäule abgegeben wird; und ersten, zweiten und dritten Bodenströmen, die von einem unteren Abschnitt der ersten Destillationssäule abgegeben werden,
Einführen des ersten Bodenstroms in eine zweite Lieferöffnung einer zweiten Destillationssäule,
Abgeben des Stroms, der in die zweite Lieferöffnung eingeführt worden ist, an jeden eines zweiten Oberstroms, der von einem oberen Abschnitt der zweiten Destillationssäule abgegeben wird; und vierten und fünften Bodenströmen, die von einem unteren Abschnitt der zweiten Destillationssäule abgegeben werden;

Wärmeaustauschen des zweiten Oberstroms mit dem dritten Bodenstrom; und
Isolieren der durch Formel 2 dargestellten Verbindung von dem oberen Abschnitt der ersten Destillationssäule und der durch Formel 1 dargestellten Verbindung von dem oberen Abschnitt der zweiten Destillationssäule, wobei Gleichungen 1 und 2 unten erfüllt werden:

[Formel 1]

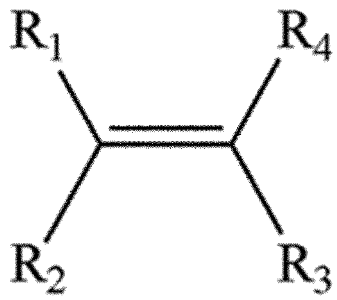

[Formel 2]

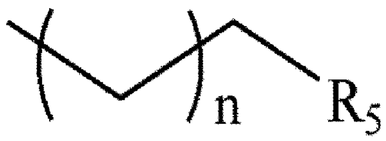

,

wobei $R_1$ eine $C_4$-$C_{12}$-Alkylgruppe ist und $R_2$ bis $R_4$ jeweils unabhängig Wasserstoff oder eine $C_4$-$C_{12}$-Alkylgruppe sind,
$R_5$ eine $C_1$-$C_4$-Alkylgruppe ist und n 1 bis 4 ist;

[Gleichung 1]

$$T_{t-2} - T_{b-3} \geq 8\ °C,$$

[Gleichung 2]

$$P_2/P_1 \geq 3{,}0,$$

wobei $T_{t-2}$ eine Temperatur des zweiten Oberstroms angibt und $T_{b-3}$ eine Temperatur des dritten Bodenstroms angibt, und
$P_1$ einen Druck des oberen Abschnitts der ersten Destillationssäule (kg/cm$^2$g) angibt und $P_2$ einen Druck des oberen Abschnitts der zweiten Destillationssäule (kg/cm$^2$g) angibt.

2. Destillationsverfahren nach Anspruch 1, wobei die durch Formel 1 dargestellte Verbindung wenigstens eine ist, ausgewählt aus der Gruppe bestehend aus 1-Octen, Isoocten und einer Mischung derselben, und wobei die durch

Formel 2 dargestellte Verbindung n-Hexan ist.

3. Destillationsverfahren nach Anspruch 2, wobei ein Gehalt von n-Hexan in dem ersten Oberstrom 90% oder mehr ist und ein Gehalt von 1-Octen, Isooctenoder einer Mischung derselben in dem zweiten Oberstrom 90% oder mehr ist.

4. Destillationsverfahren nach Anspruch 1, wobei ein Teil des zweiten Oberstroms in den Wärmeaustauscher eingeführt wird, ein Teil eines Rests des zweiten Oberstroms in den zweiten Kondensator eingeführt wird, ein Teil des zweiten Oberstroms führend durch den Wärmeaustauscher in den zweiten Kondensator eingeführt wird, und ein Teil oder alles des zweiten Oberstroms führend durch den zweiten Kondensator in den oberen Abschnitt der zweiten Destillationssäule eingeführt wird.

5. Destillationsverfahren nach Anspruch 1, wobei ein Druck des oberen Abschnitts der ersten Destillationssäule 0,05 bis 0,2 $kg/cm^2g$ ist.

6. Destillationsverfahren nach Anspruch 1, wobei ein Druck des oberen Abschnitts der zweiten Destillationssäule 1,0 bis 2,0 $kg/cm^2g$ ist.

7. Destillationsverfahren nach Anspruch 1, wobei eine Temperatur des oberen Abschnitts der ersten Destillationssäule 60 bis 80°C ist.

8. Destillationsverfahren nach Anspruch 1, wobei eine Temperatur des unteren Abschnitts der ersten Destillationssäule 120 bis 145°C ist.

9. Destillationsverfahren nach Anspruch 1, wobei eine Temperatur des oberen Abschnitts der zweiten Destillationssäule 125 bis 170°C ist.

10. Destillationsverfahren nach Anspruch 1, wobei eine Temperatur des unteren Abschnitts der zweiten Destillationssäule 130 bis 180°C ist.

11. Destillationsverfahren nach Anspruch 1, wobei die durch Formel 2 dargestellte Verbindung ein Lösungsmittel ist, das in einer Polymerisation eines Polyolefinelastomers verwendet wird.

**Revendications**

1. Procédé de distillation comprenant les étapes consistant à :

introduire une matière brute comprenant des composés représentés par les formules 1 et 2 ci-dessous et un isomère du composé dans un premier orifice d'alimentation d'une première colonne de distillation,
décharger la matière brute introduite dans chacun d'un premier courant de tête déchargé d'une section supérieure de la colonne de distillation, et les premier, deuxième et troisième courants de fond déchargés depuis une section inférieure de la première colonne de distillation,
introduire le premier courant de fond dans un deuxième orifice d'alimentation d'une deuxième colonne de distillation,
décharger le courant introduit dans le deuxième orifice d'alimentation dans chacun d'un deuxième courant de tête déchargé d'une section supérieure de la deuxième colonne de distillation ; et les quatrième et cinquième courants déchargés d'une section inférieure de la deuxième colonne de distillation ;
mettre en place un échange thermique entre le deuxième courant de tête et le troisième courant de fond ; et
isoler le composé représenté par la formule 2 de la section supérieure de la première colonne de distillation, et le composé représenté par la formule 1 de la section supérieure de la deuxième colonne de distillation,
où les équations 1 et 2 sont satisfaites :

[Formule 1]

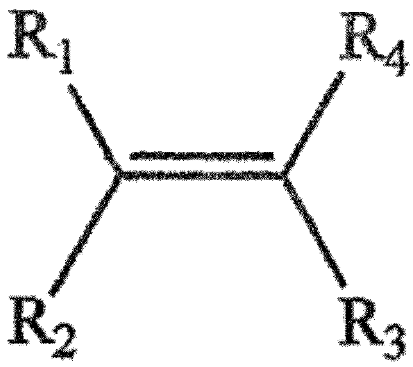

[Formule 2]

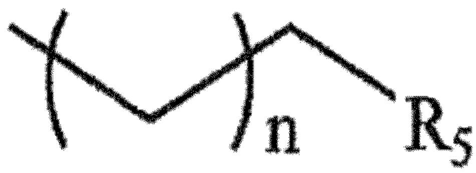

dans lesquelles formules, $R_1$ représente un groupe alkyle en $C_4$ à $C_{12}$, et $R_2$ à $R_4$ représentent chacun indépendamment l'hydrogène ou un groupe alkyle en $C_4$ à $C_{12}$,
$R_5$ représente un groupe alkyle en $C_1$ à $C_4$ et n vaut de 1 à 4 ;

[Équation 1]

$$T_{t-2} - T_{b-3} \geq 8\ ^{o}C,$$

[Équation 1]

$$P_2/P_1 \geq 3{,}0,$$

dans lesquelles $T_{t-2}$ indique une température du deuxième courant de tête, et $T_{b-3}$ indique une température du troisième courant de fond, et
$P_1$ indique une pression de la section supérieure de la première colonne de distillation ($kg/cm^2g$), et $P_2$ indique une pression de la section supérieure de la deuxième colonne de distillation ($kg/cm^2g$).

**2.** Procédé de distillation selon la Revendication 1, dans lequel le composé représenté par la formule 1 est au moins sélectionné dans le groupe constitué de 1-octène, d'iso-octène et d'un mélange de ceux-ci et le composé représenté par la formule 2 est le n-hexane.

**3.** Procédé de distillation selon la Revendication 2, dans lequel une teneur en n-hexane dans le premier courant de tête est de 90 % ou davantage, et une teneur en 1-octène, iso-octène ou un mélange de ceux-ci dans le deuxième courant de tête est de 90 % ou davantage.

**4.** Procédé de distillation selon la Revendication 1, dans lequel une partie du deuxième courant de tête est introduite dans l'échangeur thermique, une partie du reste du deuxième courant de tête est introduite dans le deuxième condensateur, une partie du deuxième courant en tête passant à travers l'échangeur thermique est introduite dans

le deuxième condensateur, et une partie de la totalité du deuxième courant de tête passant à travers le deuxième condensateur est introduite dans la section supérieure de la deuxième colonne de distillation.

**5.** Procédé de distillation selon la revendication 1, dans lequel une pression de la section supérieure de la première colonne de distillation est de 0,05 à 0,2 kg/$cm^2$g.

**6.** Procédé de distillation selon la revendication 1, dans lequel une pression de la section supérieure de la deuxième colonne de distillation et de 1,0 à 2,0 kg/$cm^2$g

**7.** Procédé de distillation selon la revendication 1, dans lequel une température de la section supérieure de la première colonne de distillation est de 60 à 80 °C.

**8.** Procédé de distillation selon la revendication 1, dans lequel une température de la section inférieure de la première colonne de distillation est de 120 à 145 °C.

**9.** Procédé de distillation selon la revendication 1, dans lequel une température de la section supérieure de la deuxième colonne de distillation est de 125 à 170 °C.

**10.** Procédé de distillation selon la revendication 1, dans lequel une température de la section inférieure de la deuxième colonne de distillation est de 130 à 180 °C.

**11.** Procédé de distillation selon la revendication 1, dans lequel le composé représenté par la formule 2 est un solvant utilisé dans la polymérisation d'un élastomère de polyoléfine.

[Figure 1]

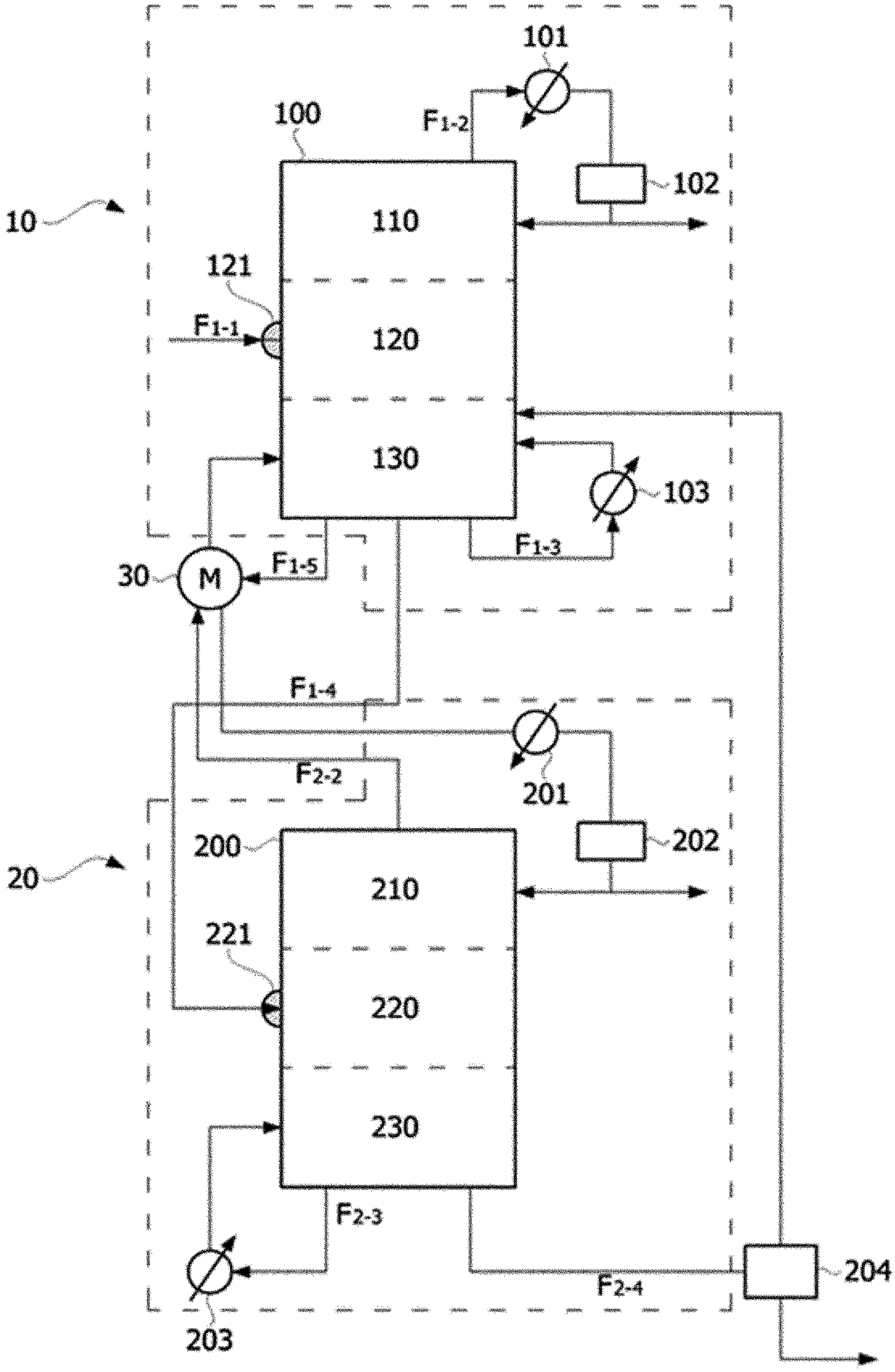
10
20
100
101
102
103
110
120
121
130
F1-1
F1-2
F1-3
F1-4
F1-5
30
M
200
201
202
203
204
210
220
221
230
F2-2
F2-3
F2-4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012026955 A1
- US 4177111 A
- US 5435436 A
- WO 9965582 A1
- US 5085740 A